(19) **Europäisches Patentamt / European Patent Office / Office européen des brevets**

(11) **EP 3 141 179 A1**

(12) # EUROPEAN PATENT APPLICATION
published in accordance with Art. 153(4) EPC

(43) Date of publication:
**15.03.2017 Bulletin 2017/11**

(21) Application number: **15878858.8**

(22) Date of filing: **11.09.2015**

(51) Int Cl.:
**A61B 1/00** *(2006.01)*  **G02B 23/24** *(2006.01)*

(86) International application number:
**PCT/JP2015/075885**

(87) International publication number:
**WO 2016/117169 (28.07.2016 Gazette 2016/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **21.01.2015 JP 2015009612**

(71) Applicant: **Olympus Corporation
Tokyo 192-8507 (JP)**

(72) Inventors:
• **NAKADE, Sho**
  **Hachioji-shi**
  **Tokyo 192-8507 (JP)**
• **MATSUDA, Eiji**
  **Hachioji-shi**
  **Tokyo 192-8507 (JP)**

(74) Representative: **Schicker, Silvia
Wuesthoff & Wuesthoff
Patentanwälte PartG mbB
Schweigerstraße 2
81541 München (DE)**

(54) **ENDOSCOPE INSERTION PART AND ENDOSCOPE**

(57) An endoscope insertion portion 2 includes: a first bending portion 7a disposed on a distal end side; a second bending portion 7b connected to a proximal end of the first bending portion 7a and provided with flexural rigidity higher than flexural rigidity of the first bending portion 7a; a tubular member 40, a first tubular part 40a of which on a distal end side disposed inside the first bending portion 7a has a bending tendency in a predetermined direction and shape-memorized, and which is provided with a second tubular part 40b on a proximal end side disposed inside the second bending portion 7b; a first wire 44 configured to move the first tubular part 40a and independently bend only the first bending portion; and second wires 47 and 48 configured to move the second tubular part 40b and independently bend only the second bending portion 7b.

**FIG. 9**

EP 3 141 179 A1

# Description

Technical Field

The present invention relates to an endoscope insertion portion including a bending portion to be bent by a hand-side operation and an endoscope.

Background Art

In recent years, medical equipment to be inserted into a subject, an endoscope for example, has been widely utilized in a medical field and an industrial field.

In particular, with an endoscope used in the medical field, by inserting an elongated insertion portion into a body cavity which is a subject, an organ inside the body cavity can be observed, and various kinds of treatments can be performed using a treatment instrument inserted into an insertion channel for the treatment instrument provided in the endoscope as needed.

For the insertion portion of such a conventional endoscope, a configuration provided with a freely bendable bending portion in order to improve insertion to the subject is well-known.

As the bending portion provided on the insertion portion of the conventional endoscope, the one for which a plurality of metallic bending pieces are freely turnably connected by rivets or the like, and the one for which a slot process is executed to a super-resilient pipe as disclosed in Japanese Patent Application Laid-Open Publication No. 2001-161631 for example in recent years are making appearance.

Such a conventional bending portion is bent by a wire which is pulled and slackened according to a hand-side operation by an operation member such as an operation lever or an operation knob provided on an operation portion.

However, the conventional endoscope has a problem that the bending portion is bent from a proximal end direction on a hand side of the bending portion when bent by pulling the wire, and it is difficult to insert an insertion portion into a complicated subject lumen or the like.

Further, in the conventional endoscope, in order to facilitate insertion when inserting the insertion portion into a complicated subject lumen or the like, it is desired to allow fine adjustment by bending only a distal end portion in a desired direction in the middle of bending the bent bending portion.

Therefore, the present invention is implemented in consideration of the above-described circumstances and an object is to provide an endoscope insertion portion and an endoscope for which a distal end portion of a bending portion can be bent in a desired bending direction and insertion of the insertion portion into a complicated subject lumen is further improved.

Disclosure of Invention

Means for Solving the Problem

An endoscope insertion portion of one aspect in the present invention includes: a first bending portion disposed on a distal end side; a second bending portion connected to a proximal end of the first bending portion and provided with flexural rigidity higher than flexural rigidity of the first bending portion; a tubular member provided inside the first bending portion and the second bending portion, a first tubular part of the tubular member on a distal end side disposed inside the first bending portion having a bending tendency in a predetermined direction and shape-memorized, and provided with a second tubular part on a proximal end side disposed inside the second bending portion; a first wire configured to move the first tubular part by pulling and slackening and independently bend only the first bending portion; and a second wire configured to move the second tubular part by pulling and slackening and independently bend only the second bending portion.

An endoscope of one aspect in the present invention includes: an endoscope insertion portion including a first bending portion disposed on a distal end side, a second bending portion connected to a proximal end of the first bending portion and provided with flexural rigidity higher than flexural rigidity of the first bending portion, a tubular member provided inside the first bending portion and the second bending portion, a first tubular part of the tubular member on a distal end side disposed inside the first bending portion having a bending tendency in a predetermined direction and shape-memorized, and provided with a second tubular part on a proximal end side disposed inside the second bending portion, a first wire configured to move the first tubular part by pulling and slackening and independently bend only the first bending portion, and a second wire configured to move the second tubular part by pulling and slackening and independently bend only the second bending portion; and an operation portion provided with an operation member configured to pull and slacken the first wire and the second wire.

According to the present invention described above, it is possible to provide an endoscope insertion portion and an endoscope for which a distal end portion of a bending portion can be bent in a desired bending direction and insertion of the insertion portion into a complicated subject lumen is further improved.

Brief Description of the Drawings

Fig. 1 is a perspective view illustrating a configuration of an endoscope of one aspect in the present invention;
Fig. 2 is a sectional view illustrating a configuration of a distal end portion of an insertion portion, of the aspect in the present invention;

Fig. 3 is a perspective view illustrating the configuration of the distal end portion of the insertion portion, of the aspect in the present invention;

Fig. 4 is a side view illustrating the configuration of the distal end portion of the insertion portion, of the aspect in the present invention;

Fig. 5 is a V-V line sectional view of Fig. 4, of the aspect in the present invention;

Fig. 6 is an operation explanatory drawing of a bending portion, a first bending portion of which is in an initial state, of the aspect in the present invention;

Fig. 7 is an operation explanatory drawing of the bending portion, the first bending portion of which is linear, of the aspect in the present invention;

Fig. 8 is an operation explanatory drawing of the bending portion, the first bending portion of which is bent downwards, of the aspect in the present invention;

Fig. 9 is an operation explanatory drawing of the entire bending portion, of the aspect in the present invention;

Fig. 10 is a side view illustrating a configuration of a treatment instrument channel in a first modification, of the aspect in the present invention;

Fig. 11 is a side view illustrating the configuration of the treatment instrument channel in a second modification, of the aspect in the present invention;

Fig. 12 is a side view illustrating the configuration of the treatment instrument channel in a third modification, of the aspect in the present invention; and

Fig. 13 is a side view illustrating the configuration of the treatment instrument channel in a fourth modification, of the aspect in the present invention.

Best Mode for Carrying Out the Invention

Hereinafter, an endoscope insertion portion of an endoscope which is the present invention will be described. Note that, in the following description, the individual drawings based on the embodiment are schematic, it should be noted that a relation between a thickness and a width of individual parts and a ratio of the thicknesses of the respective parts or the like are different from the actual ones, and even between the drawings, a part where the relation of mutual dimensions or the ratio is different is sometimes included.

Hereinafter, an endoscope including an endoscope insertion portion of one aspect of the present invention will be described based on the drawings.

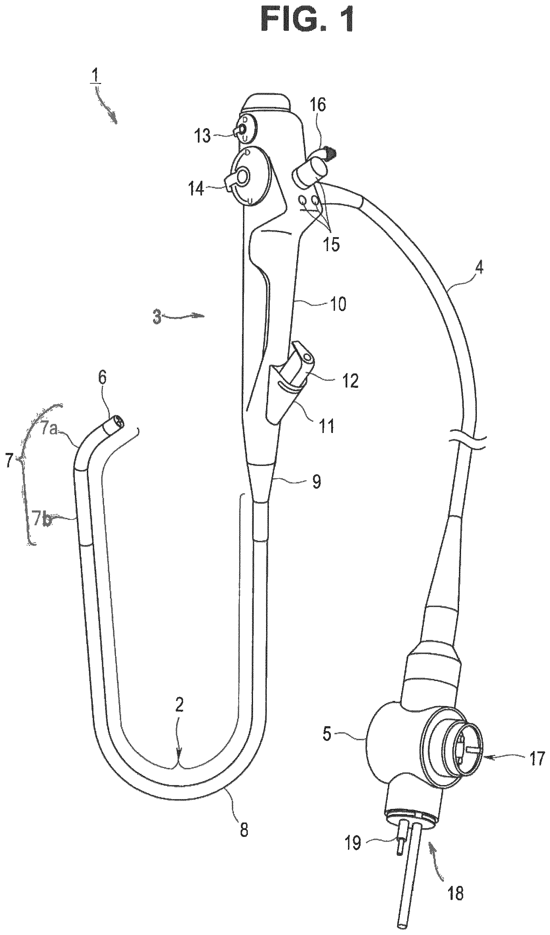

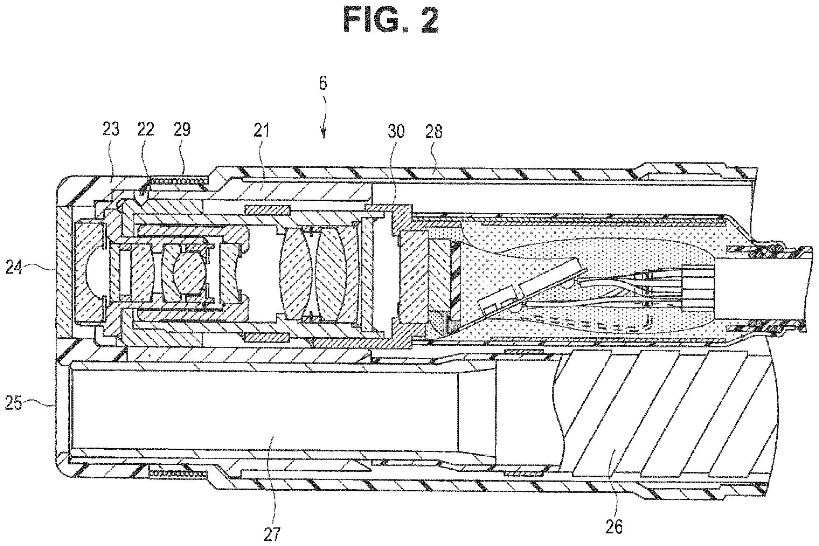

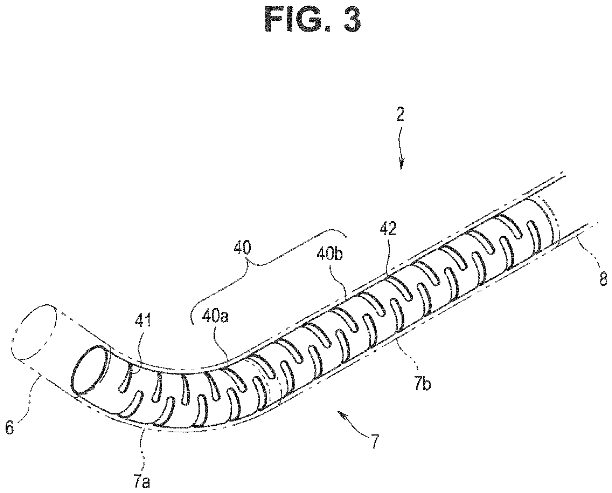

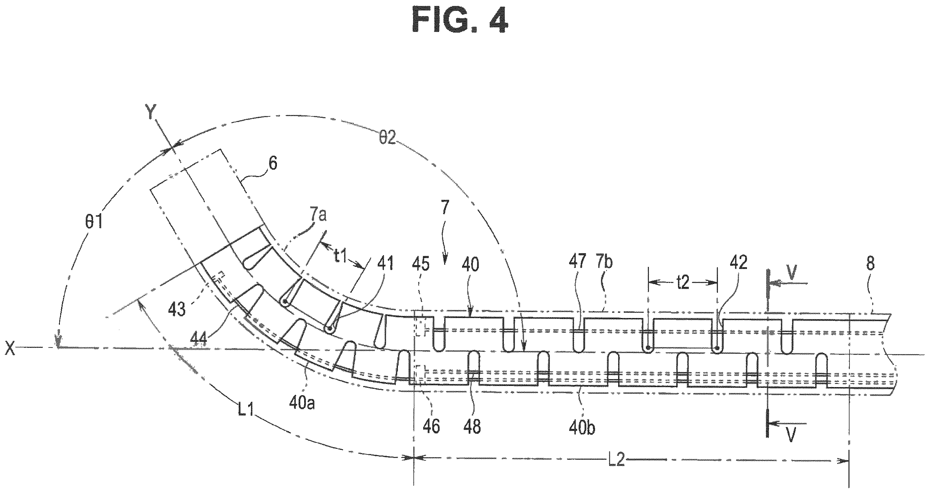

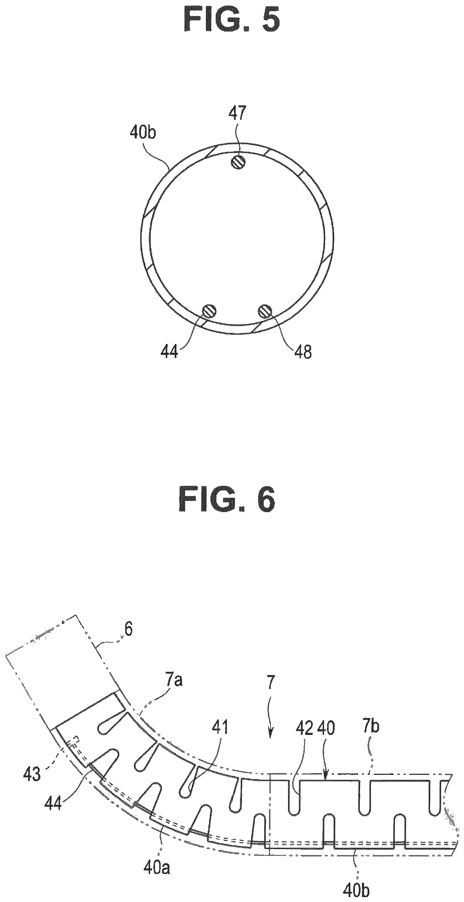

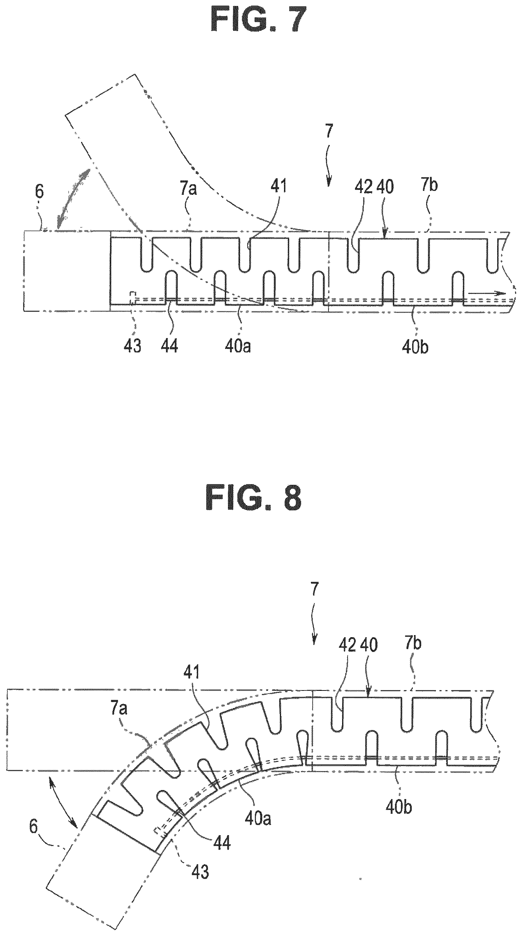

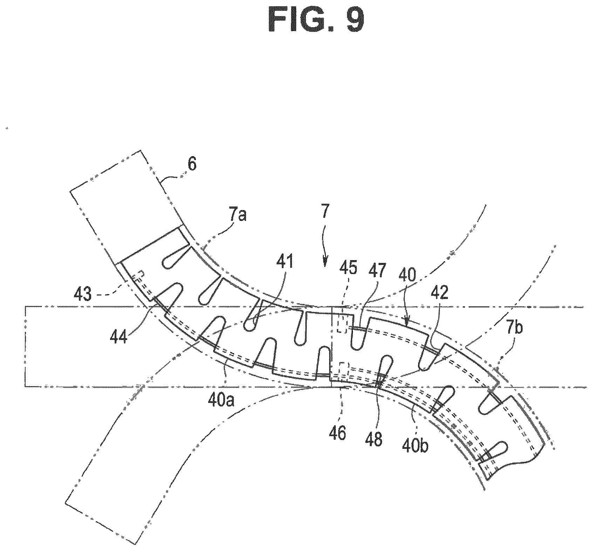

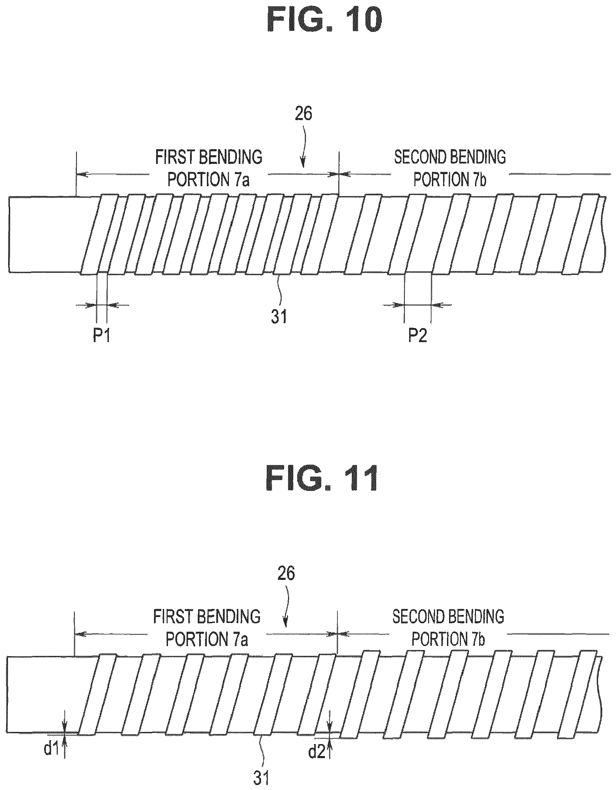

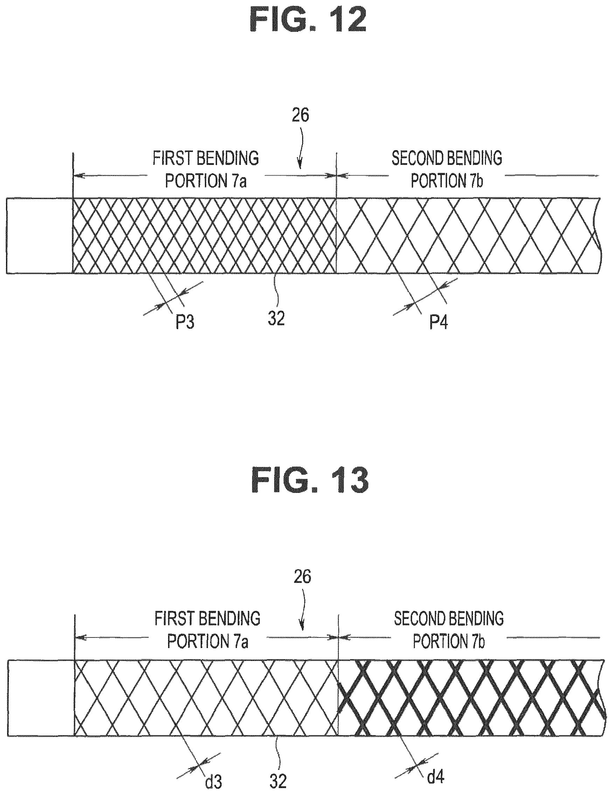

Fig. 1 to Fig. 13 relate to one aspect of the endoscope including the endoscope insertion portion of the present invention, Fig. 1 is a perspective view illustrating a configuration of the endoscope, Fig. 2 is a sectional view illustrating a configuration of a distal end portion of an insertion portion, Fig. 3 is a perspective view illustrating the configuration of the distal end portion of the insertion portion, Fig. 4 is a side view illustrating the configuration of the distal end portion of the insertion portion, Fig. 5 is a V-V line sectional view of Fig. 4, Fig. 6 is an operation explanatory drawing of a bending portion, a first bending portion of which is in an initial state, Fig. 7 is an operation explanatory drawing of the bending portion, the first bending portion of which is linear, Fig. 8 is an operation explanatory drawing of the bending portion, the first bending portion of which is bent downwards, Fig. 9 is an operation explanatory drawing of the entire bending portion, Fig. 10 is a side view illustrating a configuration of a treatment instrument channel in a first modification, Fig. 11 is a side view illustrating the configuration of the treatment instrument channel in a second modification, Fig. 12 is a side view illustrating the configuration of the treatment instrument channel in a third modification, and Fig. 13 is a side view illustrating the configuration of the treatment instrument channel in a fourth modification.

As illustrated in Fig. 1, an electronic endoscope (simply referred to as an endoscope, hereinafter) 1 of the present embodiment is mainly configured by an insertion portion 2 as an endoscope insertion portion formed in an elongated tube shape, an operation portion 3 connected to a proximal end of the insertion portion 2, a universal cord 4 which is an endoscope cable extended from the operation portion 3, and an endoscope connector 5 disposed at a distal end of the universal cord 4 or the like.

The insertion portion 2 is a tubular member which is formed by connecting a distal end portion 6, a bending portion 7, and a flexible tube portion 8 in order from a distal end side, and has flexibility. At the distal end portion 6 of the insertion portion 2, an image pickup unit which is an image pickup apparatus including image pickup means to be described later in an inside or the like is housed and arranged.

The bending portion 7 includes a first bending portion 7a on the distal end side, and a second bending portion 7b connected to a proximal end of the first bending portion 7a, and is configured to be actively bent in two up and down directions (UP-DOWN) by a turning operation of two bending levers 13 and 14 to be described later of operation members of the operation portion 3.

Note that the bending portion 7 is not limited to the one of the type, and may be the one of a type which is bent in four directions (an entire circumferential direction around an axis by up, down, right and left operations, UP-DOWN/RIGHT-LEFT) including right and left directions in addition to the up and down directions.

The flexible tube portion 8 is a tubular member formed with bendability so as to be passively flexible. Inside the flexible tube portion 8, in addition to a treatment instrument insertion channel to be described later, various kinds of signals lines extended from the image pickup unit built in the distal end portion 6 and extended further from the operation portion 3 to the inside of the universal cord 4, and a light guide for guiding illumination light from a light source unit and making the light be emitted from an illumination optical system provided in the distal end portion 6 or the like are inserted (none is shown in the figure).

The operation portion 3 includes a bend preventing portion 9 provided on the distal end side and connected with the flexible tube portion 8 covering the proximal end of the flexible tube portion 8, a grasping portion 10 connected to the bend preventing portion 9 and grasped by a hand when a user uses the endoscope 1, operation means (13, 14 and 15) to be described later, configured to operate various kinds of endoscope functions provided on an outer surface of the grasping portion 10, a treatment instrument insertion portion 11, and a suction valve 16.

Examples of the operation means provided in the operation portion 3 are, as described above, a first bending lever 13 configured to bend the first bending portion 7a of the bending portion 7, a second bending lever 14 configured to bend the second bending portion 7b of the bending portion 7, and a plurality of operation members 15 which are switches for performing respective corresponding operations of the image pickup means and illumination means or the like.

The treatment instrument insertion portion 11 is a structural member provided with a treatment instrument insertion port to insert various kinds of treatment instruments not shown in the figure and communicated with the treatment instrument insertion channel to be described later through a branching member.

At the treatment instrument insertion portion 11, a forceps plug 12 which is a lid member for opening and closing the treatment instrument insertion port and is configured to be freely attachable and detachable (exchangeable) to/from the treatment instrument insertion portion 11 is disposed.

The universal cord 4 is a composite cable configured to insert in the inside the various kinds of signal lines or the like inserted inside the insertion portion 2 from the distal end portion 6 of the insertion portion 2 to the operation portion 3 and extended further from the operation portion 3, and also insert the light guide of the light source unit not shown in the figure.

The endoscope connector 5 is configured including an electric connector portion 17 to which a signal cable connecting a video processor of external equipment not shown in the figure is connected on a side face portion, and also including a light source connector portion 18 to which a light guide bundle and an electric cable connected with the light source unit as the external equipment and not shown in the figure are connected, or the like.

Next, an internal configuration of the distal end portion 6 of the insertion portion 2 will be described based on Fig. 2.

As illustrated in Fig. 2, an image pickup unit 30 is disposed inside the distal end portion 6. The image pickup unit 30 is fitted and arranged to a distal end rigid member 21 which is a rigid distal end portion body, and is strongly fixed to the distal end rigid member 21 with a set screw 22 which is a fixing member from a side face direction together with an adhesive.

A distal end cover 23 configuring a distal end face of the distal end portion 6 is bonded and fixed so as to cover a distal end part of the distal end rigid member 21. To the distal end cover 23, an observation window 24, and an illumination window and an observation window cleaning nozzle not shown in the figure are airtightly fixed with an adhesive or screws.

Note that a distal end opening portion 25 which is a hole portion formed on the distal end cover 23 configures an opening portion of a treatment instrument channel 26 inside the distal end portion 6. The treatment instrument channel 26 is connected so as to cover a channel connection tube 27, a distal end part of which is inserted and fitted to the distal end rigid member 21.

In addition, in order to form an outer shape of the distal end portion 6 and the bending portion 7, rubber-made bending rubber 28 configured to integrally cover an outer periphery of the distal end rigid member 21 and the bending portion 7 is provided. A distal end outer peripheral portion of the bending rubber 28 is fixed to the distal end portion 6 by a yarn winding adhesion portion 29.

In addition, at the distal end rigid member 21, in addition to the image pickup unit 30 and the channel connection tube 27, the light guide configured to guide the illumination light and not shown in the figure, and a conduit or the like communicated with the observation window cleaning nozzle and a cleaning tube for cleaning the observation window of the distal end portion 6 or the like and feeding air into a body cavity are disposed.

Note that, since members such as the observation window cleaning nozzle, the cleaning tube and the light guide are conventionally well-known components, the detailed descriptions are omitted. Further, the image pickup unit 30 is also the conventionally well-known component so that the detailed descriptions are omitted.

Here, the configuration of the bending portion 7 provided in the insertion portion 2 of the endoscope 1 of the present embodiment will be described below based on Fig. 3 and Fig. 4. Note that top and bottom in the following description coincide with the up and down directions in an image for which a subject image picked up by the image pickup unit 30 is displayed on a monitor or the like, and the bending portion 7 is bent in the up and down directions by the first bending lever 13 and the second bending lever 14 provided on the operation portion 3 according to the up and down directions.

The bending portion 7 of the insertion portion 2 is, as illustrated in Fig. 3, a tubular member, and a bending pipe 40 as a bending tube here is disposed inside. The bending pipe 40 includes a first bending pipe portion 40a as a first tubular part which is disposed inside the first bending portion 7a on the distal end side and an initial position of which has a bending tendency in a predetermined direction, upwards in this case, and a second bending pipe portion 40b as a second tubular part which is disposed inside the second bending portion 7b on the proximal end side and the initial position of which is linear.

Note that the bending pipe 40 here is a member, a main body of which is a cylindrical super-resilient alloy pipe as a bending component. Examples of a super-resilient alloy material configuring the bending pipe 40 are Ni-Ti (nickel-titanium), a titanium alloy, beta titanium, pure titanium, 64 titanium, and A7075 (aluminum alloy). In addition, the bending pipe 40 may be formed by a resin pipe.

For the bending pipe 40, as illustrated in Fig. 4, in contrast with the second bending pipe portion 40b which has a longitudinal direction on an axis X on which the insertion portion 2 becomes linear, and the initial position of which is linear, as the initial position of the first bending pipe portion 40a, an axis Y in a desired direction of the distal end portion of the first bending pipe portion 40a has the upward bending tendency with a predetermined angle θ1 and shape-memorized.

That is, since the first bending pipe portion 40a disposed at the distal end portion of the bending pipe 40 has the upward bending tendency and shape-memorized, the initial position of the first bending portion 7a of the bending portion 7 is also in a state of being bent upwards.

The first bending pipe portion 40a of the bending pipe 40 has a predetermined length L1, and a plurality of first bending slots 41, a basic shape of which is a partially arcuate oblong hole extending in the circumferential direction, are provided at a predetermined interval (pitch) t1 by laser machining or the like for example.

The plurality of first bending slots 41 are formed alternately at upper and lower positions in a direction orthogonal to the longitudinal direction of the first bending pipe portion 40a.

Note that, for the first bending pipe portion 40a, the plurality of first bending slots 41 are formed in advance in a straight line state in a manufacture process, and a shape memory process of forming the upward bending tendency is executed later.

That is, the first bending pipe portion 40a is shape-memorized such that the upward bending tendency is formed in a state that the plurality of first bending slots 41 provided on an upper side are narrowed and the plurality of first bending slots 41 provided on a lower side are widened.

On the other hand, the second bending pipe portion 40b of the bending pipe 40 has a predetermined length L2 (L1<L2) longer than the predetermined length L1 of the first bending pipe portion 40a here, and similarly to the first bending pipe portion 40a, a plurality of second bending slots 42, a basic shape of which is a partially arcuate oblong hole extending in the circumferential direction, are provided at a predetermined interval (pitch) t2 by laser machining or the like for example.

The plurality of second bending slots 42 are also formed alternately at upper and lower positions in a direction orthogonal to the axis X on which the insertion portion 2 becomes linear.

Note that, by making the predetermined interval (pitch) t1 of the first bending slots 41 formed in the first bending pipe portion 40a smaller (shorter, t1<t2) than the predetermined interval (pitch) t2 of the second bending slots 42 formed in the second bending pipe portion 40b, the flexural rigidity of the first bending pipe portion 40a on the distal end side is set lower than the flexural rigidity of the second bending pipe portion 40b.

That is, the bending portion 7 of the insertion portion 2 of the present embodiment is configured such that the first bending portion 7a on the distal end side has the lower flexural rigidity (is softer) than the second bending portion 7b on the proximal end side.

Note that it is preferable to set the bending pipe 40 such that a ratio (L1:L2) of the predetermined length L1 of the first bending pipe portion 40a and the predetermined length L2 of the second bending pipe portion 40b and a ratio (θ1:θ2) of the predetermined angle θ1 of the first bending pipe portion 40a to the axis X and a maximum bending angle of the bending portion 7, a predetermined angle θ2 for which the predetermined angle θ1 is subtracted from 180° for example, coincide (L1:L2 = θ1:θ2).

The bending pipe 40 configured in this way is provided with one load generating first angle wire 44 configured to bend the first bending pipe portion 40a downwards and connected to a wire fastener 43 provided only on a lower side of an inner peripheral portion of the distal end portion of the first bending pipe portion 40a, and a pair of second angle wires 47 and 48 configured to bend the second bending pipe portion 40b in the up and down directions and connected to either one of two wire fasteners 45 and 46 provided on the top and the bottom of the inner peripheral portion of the distal end portion of the second bending pipe portion 40b.

Note that, as illustrated in Fig. 5, the first angle wire 44 and the second angle wire 48 inserted on the lower side of the bending pipe 40 are displaced and arranged in a cross sectional direction of the bending pipe 40, and mutual interference is prevented even when the predetermined length L1 of the first bending pipe portion 40a is set short.

The first angle wire 44 and the pair of second angle wires 47 and 48 are disposed inside the insertion portion 2, inserted to the operation portion 3, and pulled and slackened by the first bending lever 13 or the second bending lever 14.

Note that the first bending portion 7a of the bending portion 7 is bent by pulling and slackening the first angle wire 44 according to an operation of the first bending lever 13, and the second bending portion 7b of the bending portion 7 is bent by pulling and slackening the pair of second angle wires 47 and 48 according to an operation of the second bending lever 14.

For the bending portion 7 provided in the insertion portion 2 of the endoscope 1 configured as above, as illustrated in Fig. 6 to Fig. 8, the first bending portion 7a at the distal end portion is independently bent by pulling and slackening the first angle wire 44 by a hand-side operation by the first bending lever 13 provided on the operation portion 3.

That is, from a state of being bent upwards as the initial position illustrated in Fig. 6, when the first angle wire 44 is pulled back and a predetermined load is applied such that a lower distal end portion of the first bending pipe portion 40a is pulled to the proximal end side, the first bending portion 7a is bent to a lower side by widening of the plurality of first bending slots 41 provided on the upper side of the first bending pipe portion 40a and narrowing of the plurality of first bending slots 41 provided on the lower side, and is turned to the straight line state as illustrated in Fig. 7 for example.

In addition, from the straight line state illustrated in Fig. 7, when the first angle wire 44 is slackened and the predetermined load in a proximal end direction to the first bending pipe portion 40a is released, the first bending portion 7a returns to the state of being bent upwards as the initial position illustrated in Fig. 6 by shape memory of the first bending pipe portion 40a.

Then, from the straight line state illustrated in Fig. 7, when the first angle wire 44 is pulled back and the predetermined load is applied such that the lower distal end portion of the first bending pipe portion 40a is pulled to the proximal end side further, the first bending portion 7a is bent downwards as illustrated in Fig. 8.

Note that an operator can vary a bending state of the first bending portion 7a to a desired bending angle (state) by adjusting a pulling and slackening amount of the first angle wire 44 accompanying an operation amount of the first bending lever 13 from the state of being bent upwards in Fig. 6 to the state of being bent downwards illustrated in Fig. 8.

In this way, for the bending portion 7 of the insertion portion 2, by increasing and decreasing the load in the proximal end direction to the first bending pipe portion 40a, the bending state in the two up and down directions of the first bending portion 7a can be varied.

In addition, for the bending portion 7, since the flexural rigidity of the first bending pipe portion 40a inside the first bending portion 7a is set lower than the flexural rigidity of the second bending pipe portion 40b inside the second bending portion 7b, the first bending portion 7a is bent prior to the second bending portion 7b.

Now, in the endoscope 1, the bending portion 7 is highly frequently bent upwards when the insertion portion 2 is inserted to a subject. Therefore, the bending portion 7 here is configured such that the first bending pipe portion 40a inside the first bending portion 7a is given the bending tendency in the state of being bent upwards beforehand and shape-memorized, and the distal end portion is in the state of being bent upwards in the initial state.

In addition, for the bending portion 7, by giving the upward bending tendency to the first bending pipe portion 40a upwards and making the shape be memorized, when bending the first bending portion 7a upwards in particular, only the load to the proximal end side by the first angle wire 44 is released so that the second bending portion 7b is not bent without being affected by the load, and only the first bending portion 7a can be independently bent.

Note that it is preferable to set the bending pipe 40 to the configuration that the second bending pipe portion 40b has predetermined flexural rigidity sufficiently higher than the flexural rigidity of the first bending pipe portion 40a so that the second bending portion 7b is not bent even when the first bending portion 7a is bent downwards.

The flexural rigidity of the first bending pipe portion 40a and the flexural rigidity of the second bending pipe portion 40b can be set by adjusting the predetermined interval (pitch) $t_2$ of the second bending slots 42 formed at the second bending pipe portion 40b with respect to the predetermined interval (pitch) $t_1$ of the first bending slots 41 formed at the first bending pipe portion 40a as described above, for example.

Thus, for the bending portion 7, only the first bending portion 7a on the distal end side is independently bent without bending the second bending portion 7b on the proximal end side.

Further, for the bending portion 7, as illustrated in Fig. 9, only the second bending portion 7b on the proximal end side is independently bent by pulling and slackening the pair of second angle wires 47 and 48 connected to the two wire fasteners 45 and 46 disposed more on the proximal end side than the first bending pipe portion 40a by the hand-side operation by the second bending lever 14 provided on the operation portion 3.

Note that, even when the second bending portion 7b of the bending portion 7 is in a bent state, the operator can independently bend only the first bending portion 7a at the distal end portion of the bending portion 7 by operating the first bending lever 13 provided on the operation portion 3.

As described above, in the endoscope of the present embodiment, when bending the bending portion 7 provided in the insertion portion 2, by independently bending only the first bending portion 7a provided on the distal end side of the bending portion 7, a direction of the distal end portion 6 which is the distal end portion of the insertion portion 2 can be finely adjusted, thereby improving the insertability of the insertion portion 2.

Further, in the endoscope 1 here, even in the state of bending the second bending portion 7b of the bending portion 7, only the first bending portion 7a at the distal end portion can be bent in a desired direction and finely adjusted, and the insertion to a complicated subject lumen or the like in particular is improved.

(Modifications)

Incidentally, in the above-described bending portion 7, the flexural rigidity of the first bending portion 7a is set smaller than the flexural rigidity of the second bending portion 7b by a difference in the flexural rigidity between the first bending pipe portion 40a and the second bending pipe portion 40b of the bending pipe 40, however, instead of this or in addition to this, the flexural rigidity of the first bending portion 7a may be set to be smaller than the flexural rigidity of the second bending portion 7b by changing parameters of other built-in elements provided inside the bending portion 7.

(First Modification)

For example, the flexural rigidity of the first bending portion 7a may be set smaller than the flexural rigidity of the second bending portion 7b by gradually changing pitch widths P1 and P2 of a flex tube 31 as a protective member wound around an outer periphery of the treatment instrument channel 26 which is a tube body provided inside the bending portion 7.

Specifically, by setting the pitch width P2 of the flex tube 31 inside the second bending portion 7b larger than the pitch width P1 of the flex tube 31 provided inside the first bending portion 7a (P1<P2) and gradually changing the flexural rigidity of the treatment instrument channel 26, the flexural rigidity of the first bending portion 7a can be set to be smaller than the flexural rigidity of the second bending portion 7b.

For the flex tube 31, by reducing a spring constant k, the flexural rigidity is reduced. The flex tube 31 here is configured such that a planar body having a uniform width is wound around.

Note that the spring constant k can be calculated from the following equation (1).

$$k = Gd^4/8NaD^3 \ ... \ Equation \ (1)$$

k: spring constant
G: transverse elasticity modulus of spring member
d: wire diameter of spring
Na: effective winding number
D: coil diameter

In the above-described equation (1), by the gradual change of the pitch widths P1 and P2 of the flex tube 31, the effective winding number Na is changed. That is, since the pitch width P1 of the flex tube 31 provided inside the first bending portion 7a is smaller than the pitch width P2 of the flex tube 31 inside the second bending portion 7b (P1<P2), a denominator of the above-described equation (1) becomes large, and the spring constant k becomes small.

Therefore, by setting the pitch width P2 of the flex tube 31 inside the second bending portion 7b larger than the pitch width P1 of the flex tube 31 provided inside the first bending portion 7a (P1<P2) and gradually changing the flexural rigidity of the treatment instrument channel 26, the flexural rigidity of the first bending portion 7a can be set to be smaller than the flexural rigidity of the second bending portion 7b.

(Second Modification)

For example, the flexural rigidity of the first bending portion 7a may be set smaller than the flexural rigidity of the second bending portion 7b by gradually changing plate thicknesses d1 and d2 of the flex tube 31 as the protective member wound around the outer periphery of the treatment instrument channel 26 provided inside the bending portion 7.

Specifically, by setting the plate thickness d2 of the flex tube 31 inside the second bending portion 7b larger than the plate thickness d1 of the flex tube 31 provided inside the first bending portion 7a (d1<d2) and gradually changing the flexural rigidity of the treatment instrument channel 26, the flexural rigidity of the first bending portion 7a can be set to be smaller than the flexural rigidity of the second bending portion 7b.

In this case, by the gradual change of the pitch widths P1 and P2 of the flex tube 31, the wire diameter d of the spring in the above-described equation (1) is changed. That is, since the plate thickness d1 of the flex tube 31 provided inside the first bending portion 7a is smaller than the plate thickness d2 of the flex tube 31 inside the second bending portion 7b (d1<d2), a numerator of the above-described equation (1) becomes small, and the spring constant k becomes small.

Therefore, by setting the plate thickness d2 of the flex tube 31 inside the second bending portion 7b larger than the plate thickness d1 of the flex tube 31 provided inside the first bending portion 7a (d1<d2) and gradually changing the flexural rigidity of the treatment instrument channel 26, the flexural rigidity of the first bending portion 7a can be set to be smaller than the flexural rigidity of the second bending portion 7b.

(Third Modification)

For example, the flexural rigidity of the first bending portion 7a may be set smaller than the flexural rigidity of the second bending portion 7b by gradually changing pitch widths P3 and P4 of a braid 32 which is a metal mesh tube as a protective member put on the outer periphery of the treatment instrument channel 26 provided inside the bending portion 7.

Specifically, by setting the pitch width P4 of the braid 32 inside the second bending portion 7b larger than the pitch width P3 of the braid 32 provided inside the first bending portion 7a (P3<P4) and gradually changing the flexural rigidity of the treatment instrument channel 26, the flexural rigidity of the first bending portion 7a can be set to be smaller than the flexural rigidity of the second bending portion 7b.

(Fourth Modification)

For example, the flexural rigidity of the first bending portion 7a may be set smaller than the flexural rigidity of the second bending portion 7b by gradually changing the wire diameter of the braid 32 which is the metal mesh tube as the protective member put on the outer periphery of the treatment instrument channel 26 provided inside the bending portion 7.

Specifically, by setting the wire diameter d4 of the braid 32 inside the second bending portion 7b larger than the wire diameter d3 of the braid 32 provided inside the first bending portion 7a (d3<d4) and gradually changing the flexural rigidity of the treatment instrument channel 26, the flexural rigidity of the first bending portion 7a can be set to be smaller than the flexural rigidity of the second bending portion 7b.

Moreover, for example, in a case of a coiling treatment instrument channel 26, by changing a coil pitch or a coil wire diameter or the like for each part of the first bending portion 7a and the second bending portion 7b and gradually changing the flexural rigidity of the treatment instrument channel 26, the flexural rigidity of the first bending portion 7a can be set to be smaller than the flexural rigidity of the second bending portion 7b.

In addition, for example, in the case of a resin-made treatment instrument channel 26, by changing a proportion of a resin and a thickness of the resin or the like for each part of the first bending portion 7a and the second bending portion 7b and gradually changing the flexural rigidity of the treatment instrument channel 26, the flexural rigidity of the first bending portion 7a can be set to be smaller than the flexural rigidity of the second bending portion 7b.

Note that the modifications above are examples of setting the flexural rigidity of the first bending portion 7a to be smaller than the flexural rigidity of the second bending portion 7b by the treatment instrument channel 26 which is the built-in element provided in the bending portion 7, and the flexural rigidity of the first bending portion 7a may be set to be smaller than the flexural rigidity of the second bending portion 7b by providing an angle braid which is an exterior member provided on an inner part of the bending rubber 28 illustrated in Fig. 2 only in the second bending portion 7b.

The invention described in the above-described embodiment is not limited to the embodiment and the modifications and can be variously modified without departing from the scope in an implementation phase in addition. Further, the embodiment above includes the inventions in various stages, and various inventions can be extracted by appropriate combinations in a plurality of disclosed constituent elements.

For example, even when some constituent elements are deleted from the entire constituent elements indicated in the embodiment, in the case that the described problem can be solved and the described effect can be obtained, the configuration from which the constituent elements are deleted can be extracted as the invention.

The present application is filed claiming priority based on Japanese Patent Application No. 2015-009612 filed in Japan on January 21, 2015, and the disclosed content is incorporated in the present description, claims and drawings by reference.

**Claims**

1. An endoscope insertion portion comprising:

   a first bending portion disposed on a distal end side;
   a second bending portion connected to a proximal end of the first bending portion and provided with flexural rigidity higher than flexural rigidity of the first bending portion;
   a tubular member provided inside the first bending portion and the second bending portion, a first tubular part of the tubular member on a distal end side disposed inside the first bending portion having a bending tendency in a predetermined direction and shape-memorized, and provided with a second tubular part on a proximal end side disposed inside the second bending portion;
   a first wire configured to move the first tubular part by pulling and slackening and independently bend only the first bending portion; and
   a second wire configured to move the second tubular part by pulling and slackening and independently bend only the second bending portion.

2. The endoscope insertion portion according to claim 1,
   wherein a plurality of slots are formed in a circumferential direction on the tubular member, and
   an interval of a plurality of first slots formed at the first part is made smaller than an interval of a plurality of second slots formed at the second part, and the flexural rigidity of the second bending portion is made higher than the flexural rigidity of the first bending portion.

3. The endoscope insertion portion according to claim 2,
   wherein the plurality of slots are formed alternately at upper and lower positions in a direction orthogonal to a longitudinal direction of the tubular member.

4. The endoscope insertion portion according to any one of claim 1 to claim 3, comprising
   an image pickup apparatus configured to pick up an image of a subject,
   wherein the predetermined direction coincides with an upper direction in the image picked up by the image pickup apparatus.

5. The endoscope insertion portion according to any one of claim 1 to claim 4,
wherein a tube body is built in the first bending portion and the second bending portion, and
by gradually changing parameters such as an effective winding number and a wire diameter of a protective member provided on an outer periphery of the tube body for each part of the first bending portion and the second bending portion, the flexural rigidity of the second bending portion is made higher than the flexural rigidity of the first bending portion.

6. An endoscope comprising:

the endoscope insertion portion according to any one of claim 1 to claim 5; and
an operation portion provided with an operation member configured to pull and slacken the first wire and the second wire.

# FIG. 1

FIG. 2

# FIG. 3

FIG. 4

# FIG. 5

# FIG. 6

## FIG. 7

## FIG. 8

# FIG. 9

# FIG. 10

26

FIRST BENDING
PORTION 7a

SECOND BENDING
PORTION 7b

31

P1

P2

# FIG. 11

26

FIRST BENDING
PORTION 7a

SECOND BENDING
PORTION 7b

d1

31

d2

# FIG. 12

26

| FIRST BENDING PORTION 7a | SECOND BENDING PORTION 7b |

P3   32   P4

# FIG. 13

26

| FIRST BENDING PORTION 7a | SECOND BENDING PORTION 7b |

d3   32   d4

## INTERNATIONAL SEARCH REPORT

| International application No. |
| --- |
| PCT/JP2015/075885 |

A.   CLASSIFICATION OF SUBJECT MATTER
*A61B1/00*(2006.01)i, *G02B23/24*(2006.01)i

According to International Patent Classification (IPC) or to both national classification and IPC

B.   FIELDS SEARCHED

Minimum documentation searched (classification system followed by classification symbols)
A61B1/00, G02B23/24

Documentation searched other than minimum documentation to the extent that such documents are included in the fields searched

| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
| --- | --- | --- |
| Y | JP 2006-510463 A  (ACMI Corp.), 30 March 2006 (30.03.2006), fig. 6B, 6F, 11; paragraphs [0010], [0012], [0024] to [0025], [0036] to [0038], [0042] to [0044] & US 2006/0041188 A1 fig. 6B, 6F; paragraphs [0041], [0044], [0056] to [0058], [0069] to [0071], [0075] to [0077] | 1-6 |
| Y | JP 2-271817 A  (Olympus Optical Co., Ltd.), 06 November 1990 (06.11.1990), page 2, lower right column, line 19 to page 3, upper left column, line 5 (Family: none) | 1-6 |

| ☐   Further documents are listed in the continuation of Box C. | ☐   See patent family annex. |
| --- | --- |

| * | Special categories of cited documents: | "T" | later document published after the international filing date or priority date and not in conflict with the application but cited to understand the principle or theory underlying the invention |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 03 December 2015 (03.12.15) | 15 December 2015 (15.12.15) |

| Name and mailing address of the ISA/ | Authorized officer |
| --- | --- |
| Japan Patent Office 3-4-3,Kasumigaseki,Chiyoda-ku, Tokyo 100-8915,Japan | |
| | Telephone No. |

Form PCT/ISA/210 (second sheet) (July 2009)

**REFERENCES CITED IN THE DESCRIPTION**

**Patent documents cited in the description**

- JP 2001161631 A
- JP 2015009612 A